# EUROPEAN PATENT SPECIFICATION

(11) **EP 3 199 118 B1**
(45) Date of publication and mention of the grant of the patent: **30.12.2020**
(21) Application number: 15845124.5
(22) Date of filing: 22.09.2015
(51) Int. Cl.: A61B 18/12, A61B 18/14, A61M 25/01

(54) **CATHETER AND MANUFACTURING METHOD THEREFOR**
KATHETER UND HERSTELLUNGSVERFAHREN DAFÜR
CATHÉTER ET SON PROCÉDÉ DE FABRICATION

(30) Priority: 23.09.2014 KR 20140127193; 23.09.2014 KR 20140127194
(43) Date of publication of application: 02.08.2017
(73) Proprietor: Handok Kalos Medical Inc., Seoul 06235 (KR)
(72) Inventor: PARK, Euljoon, Seoul 06102 (KR); OH, Jungsoo, Gunpo-si Gyeonggi-do 15870 (KR); PARK, Jae Hyung, Seoul 08844 (KR); JANG, Hyunhwan, Gunpo-si Gyeonggi-do 15820 (KR); SONG, Seungwoo, Seoul 05501 (KR); WON, Jongsuk, Seongnam-si Gyeonggi-do 13610 (KR); CHO, Jiyong, Seoul 08518 (KR)
(74) Representative: Ström & Gulliksson AB
(86) International application number: PCT/KR2015/009936
(87) International publication number: WO 2016/048001

(56) References cited:
- EP-A1- 2 656 807
- JP-A- 2012 192 005
- JP-A- 2014 014 713
- JP-A- 2014 036 861
- JP-B2- 5 253 535
- KR-A- 20130 103 763
- KR-A- 20140 100 451
- US-A1- 2009 171 274

## Description

### TECHNICAL FIELD

The present disclosure relates to a catheter, and more particularly, to a medical catheter for treating diseases, especially a catheter for denervation, which ablates a part of nerves to inactivate nerve conduction, and a manufacturing method thereof.

The present application claims the benefit of Korean Patent Application No. 10-2014-0127193 filed on September 23, 2014 with and Korean Patent Application No. 10-2014-0127194 filed on September 23, 2014 the Korean Intellectual Property Office.

### BACKGROUND ART

Denervation is a surgical procedure for blocking a part of nerve paths for various nerves such as sensory nerves and automatic nerves so that stimulation or information is not delivered. The denervation is being used more and more for treatment of several diseases such as arrhythmia, pain relief, plastic surgery or the like.

In particular, as it has been recently reported that the denervation is available for treatment of hypertension, many endeavors are being made to apply the denervation for effective treatment of hypertension.

In case of hypertension, since blood pressure can be mostly controlled with drugs, most hypertensive patients are depending on drugs until now. However, if blood pressure is lowered with drugs, a hypertensive patient should take the drugs continually, which causes inconvenience and increases costs. In addition, if drugs are taken for a long time, various problems such as damage to internal organs or other side effects. Moreover, some hypertensive patients suffer from intractable hypertension which does not allow easy control of blood pressure with drugs. Since the intractable hypertension is not treated with drugs, the possibility of accidents such as a stroke, an irregular heartbeat, a kidney disease or the like increases. Therefore, the treatment of intractable hypertension is a very serious and urgent issue.

In this circumstance, the denervation attracts attention as an innovative scheme to treat hypertension. In particular, the denervation for treating hypertension may be performed by ablating sympathetic nerves around renal nerves, namely the renal artery, to inactivate nerve conduction so that the renal nerves are blocked. If the renal nerve is activated, the production of renin hormone increases by the kidney, which may cause the increase of blood pressure. Therefore, if the renal nerve is blocked, nerve conduction is not performed, and thus the hypertension may be treated, as proven by various recent experiments.

As described above, a representative renal denervation for treating hypertension is using a catheter. In the denervation using a catheter, a catheter is inserted into a part of a human body, for example the thigh, and a distal end of the catheter is located at the renal artery. In this state, heat is generated at the distal end of the catheter by means of radio frequency (RF) energy or the like to block sympathetic nerves around the renal artery.

If the denervation using a catheter is performed, a very small region is cut in a human body in comparison to the denervation using an abdominal operation. Therefore, latent complications or side effects may greatly decrease, and the time taken for treatment or recovery is very short due to local anesthesia. Therefore, the denervation using a catheter is spotlighted as a next-generation hypertension treatment method due to the above advantages.

Examples of prior art devices are presented in EP2656807A1 and JP2012192005A

However, catheter-related techniques for the application to denervation are not yet sufficiently developed and thus there is much room for improvement.

In particular, the catheter should have a very small size since it may freely move in a blood vessel. However, in an existing technique, it is very difficult to design the catheter with a small size.

Further, in catheters which have been developed or proposed, at least one electrode and various sensing devices are provided at a head portion, and also various cables for transmitting power or electric signals to the electrode and the sensing devices are provided. For this reason, it is very difficult in the existing technique to design a catheter with a small size, which includes all of the above components.

In addition, the catheter, particularly the head portion of the catheter located at a foremost location, should have a small size, and thus various structures provided thereto should also have minute sizes. However, it is not easy to handle such small structures.

Therefore, in order to make a catheter head with such minute structures, a very complicated process and high precision are demanded, and reproduction is very low. For this reason, quality and manufacturing yield of catheters are low, and the catheters may have deteriorated safety and stability.

### DISCLOSURE

### Technical Problem

The present disclosure is designed to solve the problems of the related art, and therefore the present disclosure is directed to providing a catheter having an improved head structure and its manufacturing method, which may have a small design, allow convenient production and ensure excellent reproduction.

Other objects and advantages of the present disclosure will be understood from the following descriptions and become apparent by the embodiments of the present disclosure. In addition, it is understood that the objects and advantages of the present disclosure may be implemented by components defined in the appended claims or their combinations.

### Technical Solution

In one aspect of the present disclosure, there is provided a catheter, particularly a catheter for devervation, which includes a cylinder member having a hollow formed therein; at least one electrode mounted to the cylinder member to generate heat; and a power supply wire printed on the cylinder member and connected to the electrode to give a power supply path for the electrode.

Here, two sides of the cylinder member extending from one end of the hollow to the other end thereof along a longitudinal direction of the hollow may be coupled and fixed to each other.

In addition, one of the two sides of the cylinder member may have a protrusion, the other of the two sides of the cylinder member may have an insert groove, and the protrusion may be inserted into the insert groove so that the two sides are coupled and fixed to each other.

In addition, the cylinder member may include a first cylinder having a power supply wire printed from one end thereof to the other end thereof; a second cylinder provided coaxially with the first cylinder and spaced apart from the first cylinder by a predetermined distance in the longitudinal direction of the hollow; and a connection member configured to have one end connected to the first cylinder and the other end connected to the second cylinder, the electrode being mounted to an outer surface of the connection member, the connection member having a power supply wire printed from one end thereof at least to a portion where the electrode is mounted so as to be connected to the power supply wire of the first cylinder.

In addition, when a distance between the first cylinder and the second cylinder decreases, the connection member may be at least partially bent to form a bent portion, and the bent portion moves away from the hollow.

In addition, the connection member may be provided in plural, and the electrode may be respectively mounted to at least two connection members.

In addition, at least one of the first cylinder and the second cylinder may have a step or a slope formed at a surface to which the connection member is connected, in the longitudinal direction of the hollow.

According to the invention, the electrode and the power supply wire having one end connected to the electrode are provided in plural, and the catheter further comprises a distribution unit to which at least two of the plurality of power supply wires is connected and at least one power input line is connected, so that the power supplied from a single power input line is distributed to at least two power supply wire.

Here, the distribution unit may be a multiplexer.

In addition, the cylinder member may have a cylindrical shape. According to the invention, the distribution unit is mounted to an inner wall of the cylinder member and has a curved shape corresponding to the inner wall of the cylinder member.

In addition, the distribution unit may be configured to be bendable.

In addition, the distribution unit may have a tube shape with a hollow formed therein and be coupled to one end of the cylinder member coaxially with the cylinder member.

In addition, the catheter according to the present disclosure may further include a temperature sensing member; and a temperature sensing wire printed on the cylinder member and connected to the temperature sensing member to transmit temperature information sensed by the temperature sensing member.

In addition, the catheter according to the present disclosure may further include a tactile sensing member; and a tactile sensing wire printed on the cylinder member and connected to the tactile sensing member to transmit tactile information sensed by the tactile sensing member.

In addition, the catheter according to the present disclosure may further include a shaft body formed to elongate in one direction and having an inner space formed along a longitudinal direction thereof, the shaft body being coupled to one end of the cylinder member.

In addition, the shaft body may have a power supply terminal contacting at least a part of the power supply wire printed on the cylinder member.

In addition, at least one of the cylinder member and the shaft body may include a coupling guide member configured to guide a coupling direction of the cylinder member and the shaft body.

In addition, the catheter according to the present disclosure may further include a terminal tip coupled to the other end of the cylinder member.

In another aspect of the present disclosure, there is also provided a manufacturing method of a catheter, particularly a catheter for denervation, which includes preparing a plate-shaped cylinder member; printing a power supply wire on the plate-shaped cylinder member; mounting an electrode at the plate-shaped cylinder member to be connected to the printed power supply wire; bending the cylinder member so that two sides of the cylinder member spaced apart from each other to get close to each other and thus the cylinder member has a cylinder form with a hollow therein; and coupling and fixing the two sides of the cylinder member, which have got close to each other by bending.

In another aspect of the present disclosure, there is also provided a denervation apparatus which includes the catheter according to the present disclosure.

### Advantageous Effects

According to an embodiment of the present disclosure, at least one wire is printed in a catheter, particularly in a catheter head, as an electric path. Therefore, it is not needed to separately provide a power supply cable for supplying power to an electrode. Further, it is also not needed to separately provide a sensing cable for exchanging electric signals with various sensing members, in addition to the power supply cable.

Therefore, according to an embodiment of the present disclosure, various cables present in an existing catheter head may be removed, and thus the catheter head may easily have a small design. In particular, since the catheter head is located at a front end of the catheter and allows an electrode and various sensing members to be mounted therein, the catheter head with a small design may give a great advantage.

Moreover, according to the embodiment of the present disclosure, the catheter head may easily move through a blood vessel with a small diameter, and it is also possible to prevent a wall of the blood vessel from being damaged by a moving catheter. Moreover, the present disclosure may be very easily applied to an operation in which a separate component such as a sheath is inserted into the blood vessel and then the catheter is inserted into the sheath, without directly inserting the catheter into a blood vessel.

In addition, according to an embodiment of the present disclosure, the catheter may be manufactured through a simpler process.

Further, according to an embodiment of the present disclosure, since the catheter head is prepared in advance as a wide plate-shaped two-dimensional form and then fabricated into a three-dimensional form through a bending process, the catheter may be made more simply and more easily.

In addition, according to an embodiment of the present disclosure, since the catheter may be more easily reproduced, it is possible to improve quality of the catheter, lower a defect and enhance safety and stability.

Moreover, according to an embodiment of the present disclosure, since it is not needed to insert a cable to the head portion of the catheter or connect such a cable to an electrode, the head portion of the catheter may be easily modularized.

In addition, since the catheter includes a distribution unit, it is possible to decrease the number of wires used for supplying power to various electrodes of the catheter.

Further, according to an embodiment of the present disclosure, the catheter may include various sensing wires for temperature sensing or tactile sensing in addition to the wire for power supply. In addition, even in this case, the number of sensing wires may be decreased by using the distribution unit.

Therefore, according to the embodiment of the present disclosure, since the number of power supply wires or sensing wires included in the catheter is decreased, the catheter may have a reduced diameter, which may allow the catheter to have a small design easily and also enhance safety of blood vessels of a patient.

In addition, according to the embodiment of the present disclosure, since the number of wires is decreased in most portions of the catheter other than the head portion, the catheter may be manufactured in a simple way.

Further, according to the embodiment of the present disclosure, since other components may be added as much as the space occupied by the decreased wires, it is easy to apply a new technology to the catheter.

### DESCRIPTION OF DRAWINGS

The accompanying drawings illustrate preferred embodiments of the present disclosure and, together with the foregoing disclosure, serve to provide further understanding of the technical features of the present disclosure. However, the present disclosure is not to be construed as being limited to the drawings.
Fig. 1 is a perspective view schematically showing a head of a catheter according to an embodiment of the present disclosure.
Fig. 2 is a development view showing the catheter head of Fig. 1.
Fig. 3 is a right side view showing the catheter head of Fig. 1.
Fig. 4 is a perspective view schematically showing a head of a catheter according to another embodiment of the present disclosure.
Fig. 5 is a development view showing the catheter head of Fig. 4.
Fig. 6 is a cross-sectional view, taken along the line F1-F1' of Fig. 4.
Fig. 7 shows that a connection member employed in the catheter head of Fig. 4 is bent.
Fig. 8 is a cross-sectional view, taken along the line F2-F2' of Fig. 7.
Fig. 9 is a perspective view schematically showing a head of a catheter according to another embodiment of the present disclosure.
Fig. 10 is a development view showing the catheter head of Fig. 9.
Fig. 11 is a perspective view schematically showing a head of a catheter according to another embodiment of the present disclosure.
Fig. 12 is a development view showing the catheter head of Fig. 11.
Fig. 13 is a perspective view schematically showing a head of a catheter according to another embodiment of the present disclosure.
Fig. 14 is a development view schematically showing a head of a catheter according to an embodiment of the present disclosure.
Fig. 15 is an exploded perspective view schematically showing a head of a catheter according to another embodiment of the present disclosure.
Fig. 16 is an assembled perspective view showing the catheter head of Fig. 15.
Fig. 17 is a cross-sectional view, taken along the line M-M' of Fig. 16.
Fig. 18 is a perspective view schematically showing a catheter according to another embodiment of the present disclosure.
Fig. 19 is a perspective view schematically showing a catheter according to another embodiment of the present disclosure.
Fig. 20 is a schematic flowchart for illustrating a method for manufacturing a catheter according to an embodiment of the present disclosure.

### BEST MODE

Hereinafter, preferred embodiments of the present disclosure will be described in detail with reference to the accompanying drawings. Prior to the description, it should be understood that the terms used in the specification and the appended claims should not be construed as limited to general and dictionary meanings, but interpreted based on the meanings and concepts corresponding to technical aspects of the present disclosure on the basis of the principle that the inventor is allowed to define terms appropriately for the best explanation.

The invention is set out in the appended set of claims. The embodiments and/or examples of the following description which are not conveyed by the appended claims are considered as not being part of the present invention.

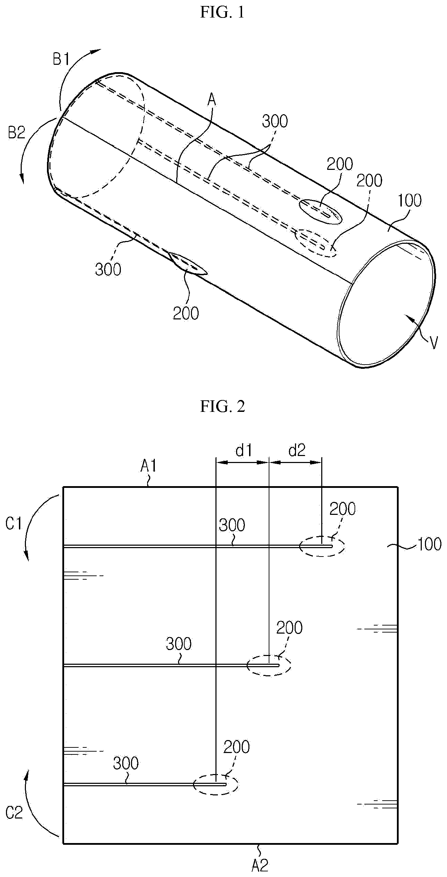

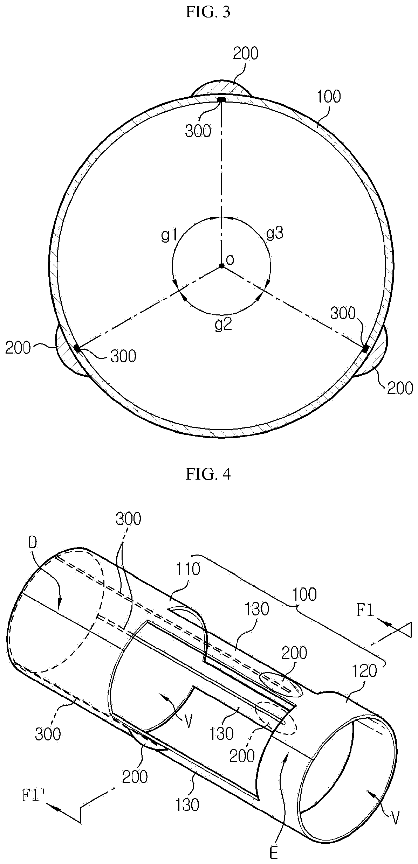

Fig. 1 is a perspective view schematically showing a head of a catheter according to an embodiment of the present disclosure, and Fig. 2 is a development view showing the catheter head of Fig. 1. In detail, Fig. 2 may be regarded as showing the portion A of Fig. 1, which is spread in directions B1 and B2. In addition, Fig. 3 is a right side view showing the catheter head of Fig. 1. However, for convenience, components not observed on the drawing are depicted with dotted lines.

Here, the head of the catheter means an end of the catheter which reaches a surgical site of a human body under a surgical procedure, between both ends of the catheter extending long in the longitudinal direction, and it may also be called a catheter tip, a catheter distal end or the like. In addition, the catheter may have a proximal end located near an operator rather than the distal end, as an end opposite to the catheter head. Hereinafter, regarding various components which are included in the catheter and extend in the longitudinal direction of the catheter to have both ends in the longitudinal direction, one end of a component located at the catheter head, namely at the distal end of the catheter, will be called a distal end of the corresponding component, and the other end of a component, located at the proximal end of the catheter, will be called a proximal end of the corresponding component.

Referring to Figs. 1 to 3, the catheter according to the present disclosure may include a cylinder member 100, an electrode 200 and a power supply wire 300.

The cylinder member 100 has a form of an elongated pipe or tube, and an empty space, namely a hollow V, is formed therein along its longitudinal direction. The hollow V may be formed so that at least one end of the hollow V along its longitudinal direction is opened. For example, in Fig. 1, the cylinder member 100 is configured so that both right and left ends of the hollow V are opened.

Meanwhile, in Fig. 1, a left end of the cylinder member 100 may be regarded as a proximal end, and a right end of the cylinder member 100 may be regarded as a distal end. A ratio of a lateral length to a vertical length of the cylinder member 100 depicted in Fig. 1 is just an example. Therefore, the cylinder member 100 may have various ratios between the lateral and vertical lengths.

The cylinder member 100 may have various shapes depending on its target or purpose and may also have various inner or outer diameters. In addition, the cylinder member 100 may be made of various materials, and the cylinder member 100 may be configured to have electric insulation as a whole in order to form a power supply wire 300 thereon.

The electrode 200 is mounted to the cylinder member 100 and may generate heat with power supplied thereto. In addition, the heat generated by the electrode 200 may ablate surrounding tissues. For example, the electrode 200 may generate heat of about 40°C or above, preferably 40°C to 80°C, to ablate nerves around a blood vessel, thereby performing denervation. However, the heat generated by the electrode 200 may have various temperatures depending on the target or purpose of the catheter.

The electrode 200 is preferably closely adhered to a blood vessel wall since the electrode 200 may contact a blood vessel wall and apply heat nerve tissues located around the blood vessel. Therefore, the electrode 200 may have a curved shape, for example with a circular, semicircular or oval section, to correspond to the inner wall of the blood vessel. In this embodiment, the electrode 200 may be adhered to the blood vessel wall in a better way and contacts the inner wall of the blood vessel with a maximum area, and thus the heat generated by the electrode 200 may be easily transferred to nerve tissues around the blood vessel. In addition, if the electrode 200 has a curved shape as described above, it is possible to prevent the inner wall of the blood vessel from being damaged by the electrode 200.

The electrode 200 may be made of platinum or stainless steel, but the electrode 200 of the present disclosure is not limited to such specific materials but may be made of various materials in consideration of various factors such as a heating method and an operation portion.

Preferably, the electrode 200 may generate heat by means of radio frequency (RF). For example, the electrode 200 may be electrically connected to a high frequency generating unit to emit high frequency energy to ablate nerves.

Meanwhile, the electrode 200 provided at the catheter may serve as a negative electrode, and a positive electrode may be connected to an energy supply unit such as a high frequency generating unit, similar to the negative electrode, and be attached to a specific portion of a human body in the form of patch or the like.

In the catheter according to the present disclosure, at least one electrode 200 may be included. In particular, as shown in the figures, a plurality of electrodes 200 may be mounted to the cylinder member 100. In this embodiment of the present disclosure, the plurality of electrodes 200 may more efficiently ablate nerves around a blood vessel.

In particular, in the catheter according to the present disclosure, the power supply wire 300 is printed on the cylinder member 100. For example, as shown in Figs. 1 to 3, the power supply wire 300 may be provided to the cylinder member 100 by placing a conductor in a two-dimensional pattern on one surface of the cylinder member 100. The power supply wire 300 may be provided to be exposed out on the surface of the cylinder member 100, but the present disclosure is not limited thereto, and the power supply wire 300 may also be buried in the cylinder member 100.

A part of the power supply wire 300 may be connected to the electrode 200 to give a power supply path for supplying power to the electrode 200. For example, as shown in Figs. 1 and 2, the power supply wire 300 may be printed to elongate from one side (a left side) to the other side (a right side) to serve as an electric circuit. In addition, if power is supplied at one end, the power may flow along the power supply wire 300 and be supplied to the electrode 200. In particular, one end of the power supply wire 300 may be connected to a high frequency generating unit so that the energy generated by the high frequency generating unit is transferred to the electrode 200. This allows the electrode 200 to generate heat with high frequency energy.

Meanwhile, even though Fig. 1 depicts that the power supply wire 300 is printed on the inner wall of the cylinder member 100, the present disclosure is not limited thereto. For example, the power supply wire 300 may also be printed on an outer wall of the cylinder member 100.

As described above, in the catheter according to the present disclosure, since the power supply wire 300 is printed on the cylinder member 100, in this configuration of the present disclosure, it is not needed that a cable for supplying power to the electrode 200 is separately provided to a head portion of the catheter. Therefore, the head portion of the catheter may have a reduced size, particularly a reduced diameter, which allows the catheter to have a small design more easily. In addition, according to the present disclosure, the electrode 200 may be mounted onto the power supply wire 300, and it is not needed to insert the electrode 200 into the hollow V of the cylinder member 100 and connect the electrode 200 to a cable. Therefore, the catheter may be manufactured more conveniently with higher reproduction, and thus the catheter may have improved safety and stability.

In addition, in the catheter according to the present disclosure, the electrode 200 may be mounted to the cylinder member 100 by being printed on the cylinder member 100. For example, the electrode 200 may be mounted to the cylinder member 100 by printing a material capable of forming an electrode on one surface of the cylinder member 100. In this embodiment of the present disclosure, the catheter may have a simple structure, easily allow a small design and be manufactured simply.

Preferably, the cylinder member 100 may be configured so that its two sides extending from one end of the hollow V to the other end thereof along a longitudinal direction of the hollow V are coupled and fixed to each other.

For example, as shown in Fig. 1, if the hollow V is formed to extend long from the left end of the cylinder member 100 the right end thereof, a coupling portion designated by A may be provided at one side of the cylinder member 100 along the longitudinal direction (right and left directions) of the hollow V.

The coupling portion may be a portion where two sides of the cylinder member 100 spaced apart from each other are coupled to each other. Therefore, if the coupling of the portion A is released and the cylinder member 100 spread along the arrows B1 and B2, the cylinder member 100 may be configured as a broad plate as shown in Fig. 2.

In other words, as shown in Fig. 2, the cylinder member 100 may be formed to have the hollow as shown in Fig. 1 if two sides A1 and A2 (Fig. 2) of a broad plate-shaped member to which the electrode 200 is mounted and on which a pattern of the power supply wire 300 is printed are coupled and fixed to each other. For this, two sides A1 and A2 to be coupled to each other encounter each other by being bent along the arrows C1 and C2, and in this state, two sides A1 and A2 may be coupled and fixed to each other.

Here, the two sides may be coupled and fixed to each other in a state where they contact each other or in a state where their surfaces partially overlap each other. In other case, the two sides may also be coupled and fixed to each other in a state where they do not contact each other but are close to each other.

In this configuration of the present disclosure, the electrode 200 may be easily mounted and the power supply wire 300 may be easily printed. In other words, as shown in Fig. 2, the cylinder member 100 according to the present disclosure has a plate shape, which is bent so that its several sides spaced apart from each other are coupled and fixed to each other, and thus it is possible to mount the electrode 200 and/or print the power supply wire 300 when cylinder member 100 is in a plate shape. Therefore, the mounting process and/or the printing process may be performed easily.

Preferably, the cylinder member 100 may have a cylindrical shape, as shown in Fig. 1.

In this configuration of the present disclosure, the plate-shaped cylinder member 100 may be more easily bent. In other words, in order to form the cylinder member 100 having a cylindrical shape, the bending process as designated by C1 and C2 in Fig. 2 may be performed just once into a curved form, and it is not needed to separately form edges. Therefore, the cylindrical shape may be formed by bending more easily.

Also preferably, two sides of the cylinder member 100 may be coupled and fixed to each other by inserting and coupling a protrusion into an insert groove. For example, in the configuration of Fig. 2, a protrusion may be formed on the upper side A1 of the plate-shaped cylinder member 100, and a groove may be formed in the lower side A2. In addition, the plate-shaped cylinder member 100 is curved into a circular shape along the arrows C1 and C2 so that the sides A1 and A2 become adjacent to each other, and then the protrusion at the upper side A1 may be inserted into the insert groove of the lower side A2 to maintain the cylindrical shape of the cylinder member 100.

Here, the coupling manner using insertion may be implemented by a hooking manner. For example, the protrusion of the upper side A1 may have a hook shape, and the protrusion may be hooked to the insert groove of the lower side A2.

However, a coupling portion of two sides of the cylinder member 100 may be fixed in various ways, without being limited to the above. For example, the two sides of the cylinder member 100 may be coupled and fixed to each other by means of an adhesive. In other words, if the plate-shaped cylinder member 100 as shown in Fig. 2 is bended in the directions of C1 and C2 so that two sides A1 and A2 encounter each other, an adhesive may be applied to any one of them so that the adhesive is interposed between the two sides A1 and A2. Therefore, in this case, the two sides A1 and A2 may keep an adhesive state by means of the adhesive.

As described above, in case of the catheter according to the present disclosure, particularly the catheter head located at the distal end of the catheter, the plate-shaped cylinder member 100 is bent and coupled to have a tube form. Therefore, the cylinder member 100 may be made of flexible material with insulation. For example, the cylinder member 100 may be made of soft material such as rubber and plastic.

Meanwhile, the catheter according to the present disclosure may include a plurality of electrodes 200. In this case, at least two electrodes 200 may be provided to be spaced apart from each other in the longitudinal direction of the cylinder member 100. For example, as indicated by d1 and d2 in Fig. 2, the plurality of electrodes 200 may be spaced apart from each other in the longitudinal direction of the cylinder member 100, namely in the longitudinal direction of the catheter.

According to this embodiment of the present disclosure, it is possible to prevent stenosis from occuring due to ablation of the plurality of electrodes. In other words, if the plurality of electrodes 200 generates heat individually, a heated portion of a blood vessel may swell into the blood vessel, and at this time, if a distance between the electrodes 200 is small in the longitudinal direction of the stenosis blood vessel, stenosis may occur. However, in this embodiment of the present disclosure, since the plurality of electrodes 200 are spaced apart from each other in the longitudinal direction of the catheter, heated portions of a blood vessel may be spaced apart from each other along the longitudinal direction of the blood vessel. Therefore, in this configuration of the present disclosure, even though heat is applied to ablate nerves around a blood vessel, it is possible to prevent stenosis from occurring at the corresponding portion.

Here, distances d1 and d2 among the electrodes 200 may be variously set depending on a catheter size or an operation region. For example, a distance between the electrodes 200 of the catheter may be 0.3 cm to 0.8 cm in the longitudinal direction of the cylinder member 100 (in the right and left direction in Fig. 1). In this embodiment, stenosis of the blood vessel may be prevented, and also it is possible to decrease a problem that nerves around a blood vessel pass between electrodes 200 and thus the nerves are not ablated by the electrodes 200, to the minimum.

Also preferably, at least two electrodes 200 may be spaced apart from each other by a predetermined angle, based on a central axis of the cylinder member 100.

For example, as shown in Fig. 3, assuming that angles formed by lines connecting from a central point O, which is a central axis of the cylinder member 100 as well as a central axis of the hollow, to each electrode 200 are respectively g1, g2 and g3, the angles g1, g2 and g3 may have an angle greater than 0°, so that three electrodes 200 are spaced apart from each other by angles. For example, the angles g1, g2 and g3 may have the same angle of 120°.

In this embodiment where the electrodes 200 are spaced apart from each other by predetermined angles based on the central axis O of the cylinder member 100, the electrodes 200 may spread widely in 360 degrees around the cylinder member 100. Therefore, regardless of locations of nerves around a blood vessel, the electrodes 200 may ablate all nerves.

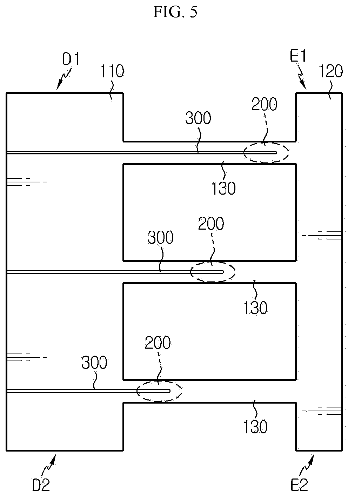

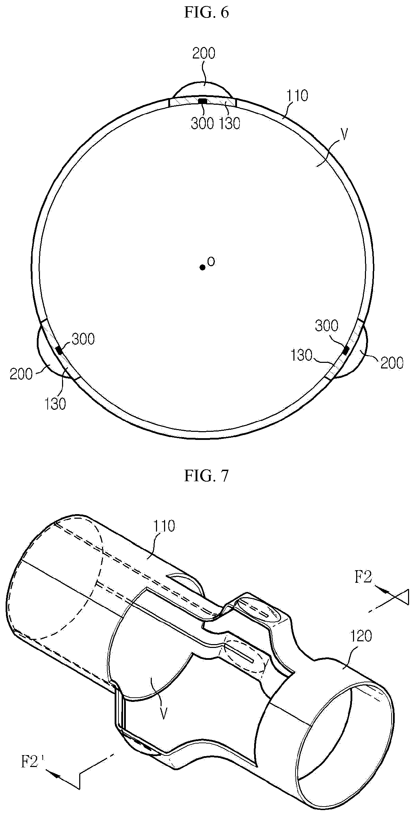

Fig. 4 is a perspective view schematically showing a head of a catheter according to another embodiment of the present disclosure, and Fig. 5 is a development view showing the catheter head of Fig. 4. In more detail, Fig. 5 may be regarded as a diagram where the portions D and E of Fig. 4 are developed. In addition, Fig. 6 is a cross-sectional view, taken along the line F1-F1' of Fig. 4. The configurations of Figs. 4 to 6, similar to those of Figs. 1 to 3, will be not described in detail here, and the following explanation will be focused only on different features.

Referring to Figs. 4 to 6, in the catheter, an electrode 200 may be mounted to a cylinder member 100, and a power supply wire 300 may be printed on the cylinder member 100 to be connected to the electrode 200, similar to the configuration of Figs. 1 to 3.

However, different from Figs. 1 to 3, the cylinder member 100 may include a first cylinder 110, a second cylinder 120 and a connection member 130.

The first cylinder 110 has a cylindrical shape with a hollow V, and the power supply wire 300 may be printed from one end of the first cylinder 110 to the other end thereof For example, in the configuration of Figs. 4 and 5, the power supply wire 300 may be printed from a left end of the first cylinder 110 to a right end thereof.

The second cylinder 120 has a hollow which is coaxial with the first cylinder 110, and may be spaced apart from the first cylinder 110 by a predetermined distance in the longitudinal direction of the hollow. For example, in the configuration of Figs. 4 and 5, the second cylinder 120 may be located at a right further to the first cylinder 110, namely at a distal end, and may be spaced apart from the first cylinder 110 by a predetermined distance.

The connection member 130 is disposed between the first cylinder 110 and the second cylinder 120 spaced apart from each other. In other words, the connection member 130 may be configured so that its one end is connected to the first cylinder 110 and the other end is connected to the second cylinder 120. For example, in the configuration of Figs. 4 and 5, a left end of the connection member 130 may be connected to the first cylinder 110, and a right end thereof may be connected to the second cylinder 120.

The connection member 130 may be integrally formed with the first cylinder 110 and/or the second cylinder 120. In this case, the connection member 130 may be prepared by cutting a broad plate-shaped material as shown in Fig. 5. In this embodiment of the present disclosure, all of the first cylinder 110, the second cylinder 120 and the connection member 130 may be prepared from a single substrate plate, and there may be no need to provide a separate coupling element between the connection member 130 and the first cylinder 110 and between connection member 130 and the second cylinder 120. Therefore, the cylinder member 100 may be manufactured in a simple way and may have a simple structure.

Meanwhile, the connection member 130 may be provided separately from the first cylinder 110 and/or the second cylinder 120. In this case, the connection member 130 may be made of material independent from the first cylinder 110 and/or the second cylinder 120. Also, in this case, both ends of the connection member 130 may be fixed to the first cylinder 110 and/or the second cylinder 120 in various ways, by using a coupling member such as a protrusion, a screw or a rivet or an adhering member.

The electrode 200 may be mounted to the surface of the connection member 130. In particular, the electrode 200 may be mounted to an outer surface of the connection member 130. Here, the outer surface of the connection member 130 means a surface located out of the cylinder member 100, rather than an inner surface forming the hollow of the cylinder member 100. If the electrode 200 is located at the outer surface of the connection member 130 as described above, the electrode 200 may be located closer to an inner wall of a blood vessel.

The power supply wire 300 may be printed on the connection member 130. In particular, the power supply wire 300 of the connection member 130 may be formed from one end of the connection member 130 to a portion where the electrode 200 is mounted. For example, as shown in Figs. 4 and 5, the power supply wire 300 may be formed to elongate from the left end of the connection member 130 to a portion where the electrode 200 is connected.

Here, the power supply wire 300 formed on the connection member 130 is connected to the power supply wire 300 of the first cylinder 110. For example, in the configuration of Figs. 4 and 5, a left end of the power supply wire 300 printed on the connection member 130 is connected to a right end of the power supply wire 300 printed on the first cylinder 110. Therefore, power supplied from one end of the first cylinder 110 may be transmitted to the electrode 200 via the power supply wire 300 of the first cylinder 110 and the power supply wire 300 of the connection member 130.

In addition, even though it is illustrated that the power supply wire 300 is printed to extend just to a portion where the electrode 200 is mounted, the present disclosure is not limited thereto. For example, in the configuration of Figs. 4 and 5, the power supply wire 300 may extend over the electrode 200 to the second cylinder 120.

Meanwhile, the first cylinder 110 and the second cylinder 120 may have coupling portions along the longitudinal direction of the hollow, as indicated by D and E in Fig. 4. In other words, the first cylinder 110 may form a coupling portion as indicated by D if two sides D1 and D2 spaced apart from each other come closer to each other by bending the cylinder member 100 and are coupled and fixed to each other. Inaddition, the second cylinder 120 may form a coupling portion as indicated by E if two sides E1 and E2 spaced apart from each other come closer to each other by bending the cylinder member 100 and are coupled and fixed to each other.

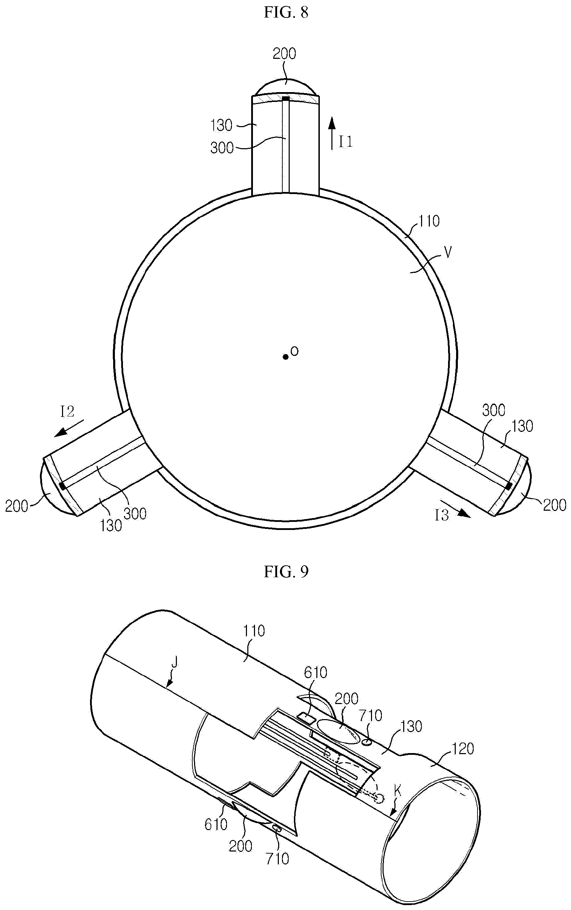

Preferably, if a distance between the first cylinder 110 and the second cylinder 120 decreases, at least a part of the connection member 130 maybe bent so that the bent portion moves away from the hollow. This will be described in more detail with reference to Figs. 7 and 8.

Fig. 7 shows that the connection member 130 employed in the catheter head of Fig. 4 is bent, and Fig. 8 is a cross-sectional view, taken along the line F2-F2' of Fig. 7.

In the configuration of Fig. 4, if a distance between the first cylinder 110 and the second cylinder 120 decreases, a distance between both ends of the connection member 130 also decreases, and thus at least a part of the connection member 130 may be bent as shown in Fig. 7.

In addition, the bent portion of the connection member 130 may move away from the hollow. Here, it may be understood that the bent portion means an apex of a bent region, namely a most bent portion in a bent region of the connection member 130, or a portion of the bent region of the connection member 130, which is farthest from the central axis of the cylinder member 100. In addition, if the bent portion is farther from the hollow, this means that bent portion is bent in an outer direction of the cylinder member 100 so that the bent portion moves away from the central axis O of the hollow.

The connection member 130 may be made of flexible material so that a bent portion is formed as a distance between the first cylinder 110 and the second cylinder 120 decreases.

Further, in an embodiment of the present disclosure, the connection member 130 may be formed from a single flexible substrate plate together with the first cylinder 110 and the second cylinder 120, and in this case, the connection member 130 may also be bendable.

However, the present disclosure is not limited thereto, and the connection member 130 may also be made of material different from those of the first cylinder 110 and the second cylinder 120. For example, the connection member 130 may have a severe bent in comparison to the first cylinder 110 and the second cylinder 120 and thus may be made of material with greater flexibility, for example greater elongation, in comparison to the first cylinder 110 and the second cylinder 120.

Meanwhile, referring to Fig. 6, in the present disclosure, the connection member 130 may be configured so that its vertical section perpendicular to the longitudinal direction is bent based on the central axis O of the hollow, similar to the first cylinder 110 and the second cylinder 120. Therefore, if a distance between both ends thereof decreases, the connection member 130 may be bent so that the bent portion moves away from the central axis O of the hollow, as indicated by the arrows I1, I2 and I3 in Fig. 8.

The electrode 200 may be provided at the bent portion of the connection member 130. At this time, the electrode 200 may be provided at a location farthest from the central axis of the hollow, in the bent portion of the connection member 130. In other words, when the first cylinder 110 and the second cylinder 120 get closer to form the bent portion at the connection member 130, the electrode 200 may be provided at an apex of the bent portion, which is located farthest from the central axis of the hollow.

In this embodiment, while the catheter head is being moved to an operation region, the catheter may move as shown in Fig. 4, but when the catheter head reaches the operation region, the connection member 130 may be bent as shown in Fig. 7. If so, the electrode 200 may protrude from the cylinder member 100 to the maximum, so that the electrode 200 may be closer to a blood vessel wall. In addition, it is possible to prevent the electrode 200 from protruding as above while it is moving. In this case, the catheter head may move more easily, and it is possible to prevent the blood vessel wall from being damaged by the connection member 130, the electrode 200 or the like.

Preferably, as shown in Figs. 4 to 8, the cylinder member 100 may include a plurality of connection members 130. Also, in this case, the electrodes 200 may be mounted to two or more connection members 130, respectively.

For example, as shown in Fig. 4 to 8, the cylinder member 100 may include three connection members 130. Also, three connection members 130 may be respectively provided with electrodes 200. At this time, in order to supply power to the three electrodes 200, each of three connection members 130 may have a power supply wire 300 printed thereon, and corresponding to such three power supply wires 300, three power supply wires 300 may be printed on the first cylinder 110.

If a plurality of connection members 130 are provided and also a plurality of electrodes 200 are provided accordingly, as described above, nerves around a blood vessel may be ablated more efficiently.

In this configuration, at least two connection members 130 may be configured so that mounting points of the electrodes 200 are spaced apart from each other by a predetermined distance in the longitudinal direction of the hollow. In this case, the electrodes 200 may be spaced apart from each other in the longitudinal direction of the hollow, namely in the longitudinal direction of the catheter. Therefore, according to this embodiment of the present disclosure, as described in the former embodiment depicted in Figs. 1 to 3, it is possible to prevent stenosis and enhance a denervation ratio by using the plurality of electrodes 200.

Also, in this configuration, at least two connection members 130 may be spaced apart from each other by a predetermined angle, based on the central axis in the longitudinal direction of the hollow. For example, as shown in Figs. 4 and 6, the connection members 130 may be configured to be spaced apart from each other in a radial direction, based on the central axis of the hollow, for example by an angle of 120°. According to this embodiment of the present disclosure, as described above in the former embodiment depicted in Figs. 1 to 3, it is possible to prevent stenosis and enhance a denervation effect by using the plurality of electrodes 200.

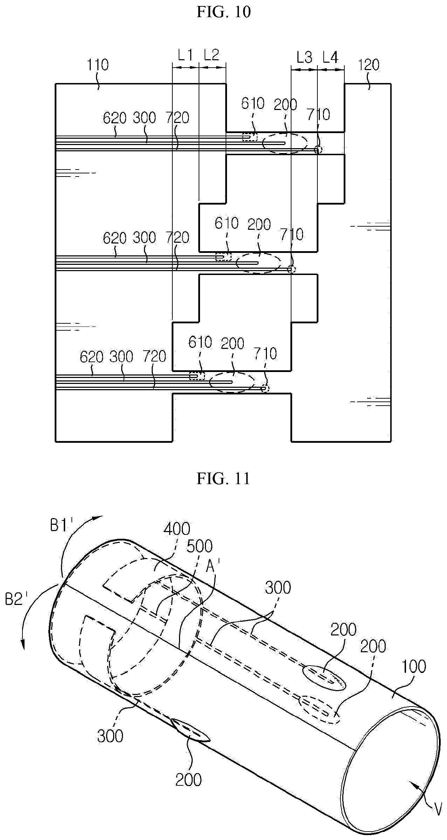

Fig. 9 is a perspective view schematically showing a head of a catheter according to another embodiment of the present disclosure, and Fig. 10 is a development view showing the catheter head of Fig. 9. In more detail, Fig. 10 may be regarded as a diagram where the portions J and K of Fig. 9 are sperated and developed. The configurations of Figs. 9 and 10, similar to those of Figs. 1 to 8, will be not described in detail here, and the following explanation will be focused only on different features.

Referring to Figs. 9 to 10, the cylinder member 100 may include a first cylinder 110, a second cylinder 120 and a plurality of connection members 130. In addition, the first cylinder 110 and the second cylinder 120 may have coupling portions as indicated by J and K.

In particular, in the configuration of Figs. 9 and 10, at least two connection members 130 may be configured so that their connection points to the first cylinder 110 and/or the second cylinder 120 are spaced apart from each other by a predetermined distance in the longitudinal direction of the hollow.

In more detail, as shown in Fig. 10, when three connection members 130 are provided at the cylinder member 100, these connection members 130 may configured so that left ends thereof connected to the first cylinder 110 are spaced apart from each other by predetermined distances of L1 and L2. In addition, in the configuration depicted in Fig. 10, the connection members 130 may be configured so that their right ends connected to the second cylinder 120 are spaced apart from each other by predetermined distances of L3 and L4.

As described above, so that the connection members 130 are connected to the first cylinder 110 and/or the second cylinder 120 at spaced points, at least one of the first cylinder 110 and the second cylinder 120 may have a step formed on its surface connected to the connection member 130 as shown in Figs. 9 and 10. For example, if three connection members 130 are connected to a right surface of the first cylinder 110, three stages having steps in a lateral direction may be formed at the right surface of the first cylinder 110. In addition, at a left surface of the second cylinder 120 connected to three connection members 130, three stages with such lateral steps may also be formed.

As described above, if the connection members 130 are connected to the first cylinder 110 and/or the second cylinder 120 at points spaced apart from each other by a predetermined distance in the longitudinal direction of the hollow, when the first cylinder 110 and the second cylinder 120 move closer to each other to bend the connection members 130, the bent portions may be spaced apart from each other by a predetermined distance. In other words, when both ends move closer to bend the connection member 130, the bent portion may be easily formed at the center of the connection member 130. In the embodiment of the present disclosure, central portions of the connection members 130 may be spaced apart from each other by a predetermined distance in the longitudinal direction of the catheter, and thus the bent portions of the connection members 130 may be spaced apart from each other by a predetermined distance. Therefore, as long as each electrode 200 is mounted to the central portion of each connection member 130, when the connection members 130 are bent, the electrodes 200 may be easily spaced apart from each other by a predetermined distance.

Meanwhile, even though Figs. 9 and 10 illustrate that the first cylinder 110 and the second cylinder 120 have steps so that connection points of the connection members 130 are spaced apart from each other by a predetermined distance, but the present disclosure is not limited thereto.

As another example, the first cylinder 110 and/or the second cylinder 120 may have a slope at a surface thereof connected to the connection members 130, so that connection points of the connection members 130 are spaced apart from each other by a predetermined distance.

In addition, in the catheter according to the present disclosure, the connection points of the connection members 130 to the first cylinder 110 and the second cylinder 120 may be spaced apart from each other in various ways.

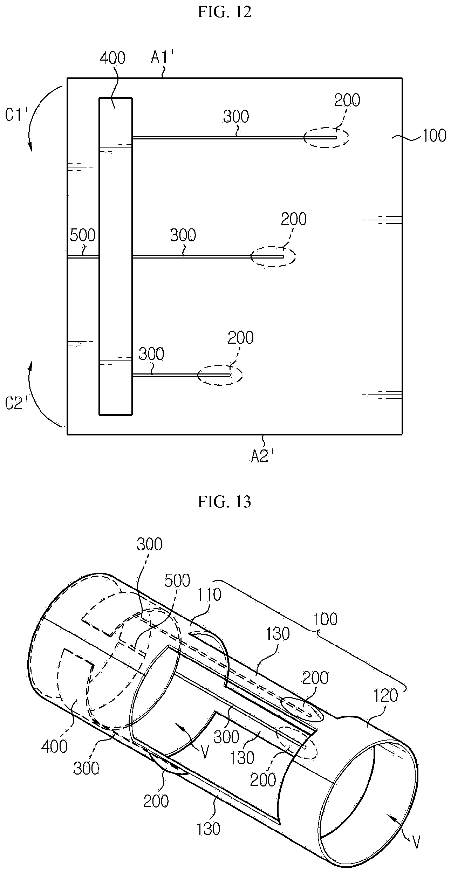

Fig. 11 is a perspective view schematically showing a head of a catheter according to another embodiment of the present disclosure, and Fig. 12 is a development view showing the catheter head of Fig. 11. In more detail, Fig. 12 may be regarded as a diagram where the portion A' of Fig. 11 is separated and developed in the directions B1' and B2'. The features of this embodiment, similar or identical to those of the former embodiments, will be not described in detail here, and the following explanation will be focused only on different features.

Referring to Figs. 11 and 12, the catheter according to the present disclosure may further include a distribution unit 400. In particular, the catheter according to the present disclosure may include a plurality of electrodes and a plurality of power supply wires, and in this case, the distribution unit 400 may be further included.

The distribution unit 400 may distribute the power supplied from a single power input line 500 to at least two power supply wires 300.

For this, the distribution unit 400 may be connected to at least two power supply wires 300, among a plurality of power supply wires 300. In addition, the distribution unit 400 may be connected to at least one power input line 500.

For example, as shown in Figs. 11 and 12, the distribution unit 400 may be connected to left ends of three power supply wires 300 and a right end of a single power input line 500. In this case, the power supplied from the single power input line 500 may be distributed to the three power supply wires 300, respectively, by the distribution unit 400.

In the catheter according to this embodiment of the present disclosure, the number of power supply wires 300 may be reduced, and thus it is possible to reduce a diameter of the catheter and simplify a manufacturing process of the catheter. For example, in the configuration of Figs. 11 and 12, in order to supply power to three electrodes 200, three lines are provided just to a right portion of the distribution unit 400, and only a single line is provided to a left portion of the distribution unit 400. Therefore, the left portion of the distribution unit 400 may have a reduced diameter, and another component may be added thereto as much as the space occupied by the reduced number of lines.

In particular, the configuration depicted in Fig. 11 is the head portion of the catheter, and a catheter may have a much greater length at a proximal end of the catheter head, namely at a left end thereof, for example by coupling a shaft body explained below thereto. In this case, since the shaft body may have just a single line for power supply, the shaft body may have a reduced diameter as a whole, which may be understood as the catheter may mostly have a smaller size.

Preferably, the distribution unit 400 may be implemented by using a multiplexer. Here, the multiplexer may be defined as a device having different numbers of input lines and output lines to multiplex and distribute a single power or electric signal or select one of a plurality of powers or electric signals.

In particular, in the present disclosure, the distribution unit 400 may be configured to perform both a multiplexer of giving a single output from a plurality of inputs, in a narrow sense, and a de-multiplexer for giving a plurality of outputs from a single input, in a narrow sense.

Preferably, the cylinder member 100 may have a cylindrical shape. In other words, as shown in Fig. 11, the cylinder member 100 may have a cylindrical shape with a circular section in a direction perpendicular to the central axis of the hollow.

In this configuration, the distribution unit 400 may be mounted to an inner wall of the cylinder member 100. In particular, since the cylinder member 100 having a cylindrical shape has an inner wall with a curved surface, the distribution unit 400 may have a curved shape corresponding to the inner surface of the cylinder member 100 as shown in Fig. 11.

In this configuration of the present disclosure, since the distribution unit 400 is closely adhered to the inner surface of the cylinder member 100, it is possible to reduce the space occupied by the distribution unit 400 and thus decrease a diameter of the cylinder member 100. In addition, in this configuration of the present disclosure, another component may be present or move in the hollow of the cylinder member 100, and at this time, it is possible to minimize that the distribution unit 400 disturbs the presence or movement of such a component.

Meanwhile, in the embodiment of Figs. 11 and 12, two sides A1' and A2' (Fig. 12) of the cylinder member 100 spaced apart from each other may be coupled and fixed with respect to a broad plate-shaped member to have a hollow, similar to the former embodiment of Figs. 1 and 2. For this, two sides A1' and A2' to be coupled to each other may encounter each other by being bent in directions designated by the arrows C1' and C2', and two sides encountering each other may be coupled and fixed to each other.

In this configuration, the distribution unit 400 may be configured to be bendable. In other words, if the cylinder member 100 is transformed from a plate state into a cylindrical shape by bending as in this embodiment, the distribution unit 400 mounted to the cylinder member 100 may be made of flexible material to be bent from a planar shape into a curved shape.

In this configuration of the present disclosure, as shown in Fig. 12, if the distribution unit 400 is mounted to the plate-shaped cylinder member 100 before the cylinder member 100 is bent and then the cylinder member 100 is bent in the direction indicated by the arrows C1' and C2', it is possible to prevent the distribution unit 400 from being damaged by such bending. In addition, in this configuration, the catheter may be manufactured more easily. Moreover, the distribution unit 400 may be closely adhered to the inner surface of the cylinder member 100 more easily, which may reduce a diameter of the cylinder member 100.

As described above, in order to make the distribution unit 400 bendable, the distribution unit 400 may be configured with a plate or sheet form to have as broad area as possible.

In particular, as shown in Fig. 12, the distribution unit 400 may be configured to be longer in the bending direction (a vertical direction in Fig. 12) of the cylinder member 100 in comparison to the longitudinal direction (a lateral direction in Fig. 12) of the central axis of the hollow.

Moreover, as shown in Fig. 11, the cylinder member 100 may have a cylindrical shape. In this case, plate-shaped cylinder member 100 may be bent more easily, and it would be easier to bend the distribution unit 400 or prevent the distribution unit 400 from being damaged.

Fig. 13 is a perspective view schematically showing a catheter according to another embodiment of the present disclosure.

The catheter depicted in Fig. 13 is similar to the catheter of Fig. 4, except that a distribution unit 400 and a power input line 500 are additionally included. Referring to Fig. 13, the distribution unit 400 may be mounted to the first cylinder 110. Therefore, three power supply wires 300 may be formed at a distal end of the distribution unit 400, namely a right side thereof and a single power input line 500 may be formed at a left side of the distribution unit 400.

Preferably, the catheter according to the present disclosure may further include a temperature sensing member 610 and a temperature sensing wire 620, as shown in Figs. 9 and 10.

The temperature sensing member 610 is a component for measuring temperature of the surrounding. For example, the temperature sensing member 610 may be a thermocouple. In particular, the temperature sensing member 610 may be mounted around the electrode 200.

According to this embodiment of the present disclosure, since the temperature sensing member 610 may measure temperature of the surrounding it is possible to check whether the heat emitted from the electrode 200 is suitable for ablating nerve tissues around a blood vessel or excessively high or low.

In particular, in the disclosure head of the catheter according to an embodiment of the present disclosure, the electrode 200 is provided at the connection member 130, and the connection member 130 may be bent in a direction in which the bent portion moves away from the central axis of the catheter head during a surgical operation. Therefore, since the temperature sensing member 610 is also provided at the connection member 130, similar to the electrode 200, the quantity of heat by the electrode 200 may be measured more accurately.

Further, if a plurality of connection members 130 is provided, it is also possible to provide a plurality of temperature sensing members, which are mounted to different connection members 130.

The temperature sensing wire 620 may be printed on the cylinder member 100 as a two-dimensional circuit pattern, similar to the power supply wire 300. For example, as shown in Figs. 9 and 10, if the cylinder member 100 includes the first cylinder 110, the second cylinder 120 and the connection member, the temperature sensing wire 620 may be formed to extend from a left end of the first cylinder 110 to a right end thereof and also further elongate therefrom from a left end of the connection member 130 to a portion where the temperature sensing member 610 is mounted. In addition, the temperature sensing wire 620 may not be electrically connected to the power supply wire 300 but separated therefrom.

The temperature sensing wire 620 may be partially connected to the temperature sensing member 610 to give a path for transmitting temperature information sensed by the temperature sensing member 610. For example, if the temperature sensing member 610 is configured with a thermocouple, the current generated by the thermocouple may be transmitted through the temperature sensing wire 620 to an external temperature measuring device connected to the catheter.

Preferably, the catheter according to the present disclosure may further include a tactile sensing member 710 and a tactile sensing wire 720, as shown in Figs. 9 and 10.

The tactile sensing member 710 is a component for measuring tactile information. The tactile sensing member 710 may be mounted in or around the electrode 200. In this case, the tactile sensing member 710 may check whether the electrode 200 is in contact with a blood vessel wall.

According to this embodiment of the present disclosure, if it is checked by the tactile sensing member 710 that the electrode 200 is in contact with the blood vessel wall, power may be supplied to the electrode 200 to generate heat by the electrode 200. In addition, a distance between the first cylinder 110 and the second cylinder 120 may be controlled by means of the information obtained by the tactile sensing member 710. For example, according to an embodiment of the present disclosure, if the distance between the first cylinder 110 and the second cylinder 120 decreases, the connection member 130 is bent so that the electrode 200 moves closer to a blood vessel, and thus the distance between the first cylinder 110 and the second cylinder 120 may be reduced until the tactile sensing member 710 checks that the electrode 200 is in contact with the blood vessel wall.

Meanwhile, even though Figs. 9 and 10 depict that the tactile sensing member 710 is mounted around the electrode 200, the tactile sensing member 710 may also be mounted in the electrode 200. In this case, the tactile sensing member 710 may give more accurate information about whether the electrode 200 is in contact with the blood vessel wall.

The tactile sensing wire 720 may be printed as a two-dimensional circuit pattern on the cylinder member 100, similar to the power supply wire 300. For example, as shown in Figs. 9 and 10, if the cylinder member 100 includes the first cylinder 110, the second cylinder 120 and the connection member 130, the tactile sensing wire 720 may be formed to extend from a left end of the first cylinder 110 to a right end thereof, and further elongate therefrom from a left end of the connection member 130 to a portion where the tactile sensing member 710 is mounted.

The tactile sensing wire 720 may be partially connected to the tactile sensing member 710 to give a path for transmitting tactile information sensed by the tactile sensing member 710.

The tactile sensing wire 720 may not be electrically connected to the power supply wire 300 but separated therefrom. In addition, if the temperature sensing wire 620 is formed at the cylinder member 100, the tactile sensing wire 720 may be formed to be separated from the temperature sensing wire 620. In this case, as shown in Figs. 9 and 10, three wires, namely a power supply wire 300, a temperature sensing wire 620 and a tactile sensing wire 720, may be provided to a single connection member 130. Further, as shown in Figs. 9 and 10, if three connection members 130 are provided at the cylinder member 100, nine wires may be provided in total.

Meanwhile, the catheter according to the present disclosure may further include various sensing members in addition to the temperature sensing member 610 and the tactile sensing member 710, and wire patterns for exchanging signals with such sensing members may be further printed on the cylinder member 100.

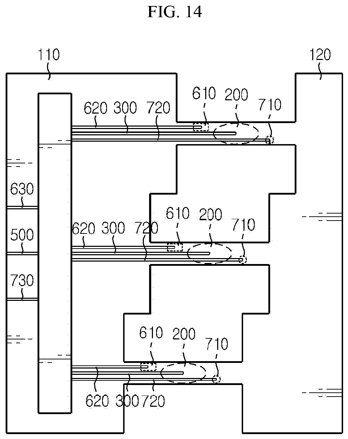

In the catheter including the temperature sensing wire 620 and/or the tactile sensing wire 720, if a distribution unit 400 is further included, the temperature sensing wire 620 and/or the tactile sensing wire 720 may be connected to the distribution unit 400 together with the power supply wire 300. This will be described in more detail with reference to Fig. 14.

Fig. 14 is a development view schematically showing a head of a catheter according to an embodiment of the present disclosure. The configuration of Fig. 14 will be explained based on features different from the former embodiments, particularly the embodiment of Fig. 10.

Referring to Fig. 14, a plurality of temperature sensing wires 620 may be provided, and proximal ends of at least two temperature sensing wires 620 among them may be connected to the distribution unit 400. In this case, a single temperature output line 630 for transmitting temperature information sent from at least two temperature sensing wires 620 may be connected to the distribution unit 400.

In particular, in the configuration of Fig. 14, three temperature sensing wires 620 and a single temperature output line 630 are connected to the distribution unit 400. In this case, the distribution unit 400 may output temperature information transmitted from three temperature sensing wire 620 to a single temperature output line 630.

Therefore, according to this embodiment of the present disclosure, the number of temperature output lines 630 for transmitting temperature information may be reduced to a most region of the catheter located at a proximal end of the distribution unit 400, and thus the catheter may be manufactured with a smaller design, have a simplified structure and be manufactured conveniently.

In addition, a plurality of tactile sensing wires 720 may be provided, and proximal ends of at least two tactile sensing wires 720 of them may be connected to the distribution unit 400. In this case, a single tactile output line 730 for transmitting tactile information sent from at least two tactile sensing wires 720 may be connected to the distribution unit 400.

For example, in the configuration of Fig. 14, three tactile sensing wires 720 and a single tactile output line 730 are connected to the distribution unit 400. In this case, the distribution unit 400 may output tactile information transmitted from three tactile sensing wires 720 to the single tactile output line 730.

Therefore, according to this embodiment of the present disclosure, the number of tactile output lines 730 for transmitting tactile information may be reduced to a most region of the catheter located at a proximal end of the distribution unit 400, and thus the catheter may be manufactured with a smaller design, have a simplified structure and be manufactured conveniently.

In particular, even though a plurality of electrodes 200, temperature sensing members 610 and tactile sensing members 710 are provided at the catheter, in the present disclosure, the number of lines for supplying power thereto or transmitting electric signals may be greatly reduced by means of the distribution unit 400, which may be advantageous in designing the catheter smaller and simplifying its structure.

For example, if three power supply wires 300, three temperature sensing wires 620 and three tactile sensing wires 720 are provided as shown in Fig. 14, nine lines may be provided at the head portion of the catheter in total. However, in the present disclosure, the nine lines may be greatly reduced into three lines by means of the distribution unit 400.

Meanwhile, the power input line 500, the temperature output line 630 and the tactile output line 730 may be configured with cables, but they may also be formed by printing conductors on the cylinder member, similar to the power supply wire, the temperature sensing wire and the tactile sensing wire.

Preferably, the catheter according to the present disclosure may further include a shaft body.

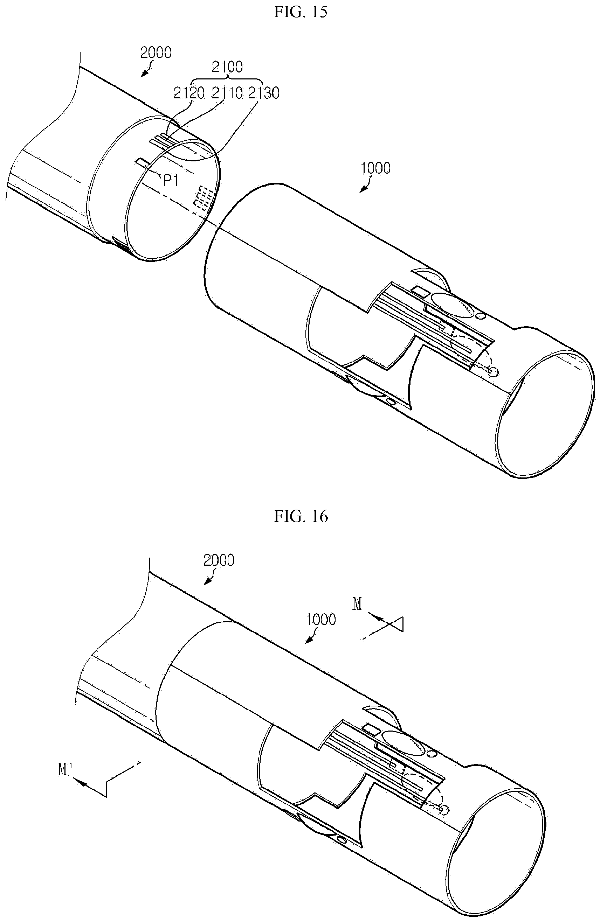

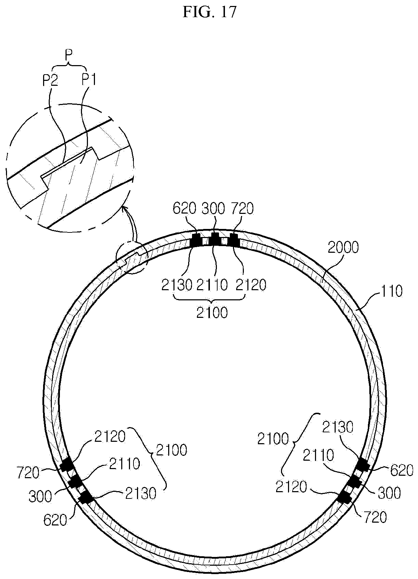

Fig. 15 is an exploded perspective view schematically showing a head of a catheter according to another embodiment of the present disclosure, and Fig. 16 is an assembled perspective view showing the catheter head of Fig. 15. In addition, Fig. 17 is a cross-sectional view, taken along the line M-M' of Fig. 16.

Referring to Figs. 15 to 17, the catheter according to the present disclosure includes the cylinder member 100, the electrode 200 and the power supply wire 300, described above, at the catheter head 1000, which may be located at a distal end of the catheter. In addition, the catheter according to the present disclosure may further include a shaft body 2000 coupled to a proximal end of the catheter head 1000.

The shaft body 2000 is coupled to the proximal end of the cylinder member 100 in various ways. For example, as shown in Fig. 15, a distal end of the shaft body 2000 may be configured to be inserted into the hollow of the cylinder member 100. In other case, the catheter head and the shaft body may be coupled so that the proximal end of the catheter head is inserted into the distal end of the shaft body.

In particular, the shaft body 2000 may include a connection terminal 2100 at a distal end thereof so that the shaft body 2000 may be coupled to various wires provided at the catheter head, when being coupled to the catheter head.

For example, as shown in Figs. 15 to 17, the power supply wire 300, the temperature sensing wire 620 and/or the tactile sensing wire 720 may be printed on the inner surface of the cylinder member 100 of the catheter head. In addition, the shaft body 2000 may further include a power supply terminal 2110 connected to the power supply wire 300, a temperature sensing terminal 2120 connected to the temperature sensing wire 620 and/or a tactile sensing terminal 2130 connected to the tactile sensing wire 720, at an outer surface of the distal end.

The terminal of the shaft body may be implemented in various ways by printing a conductor on the surface of the shaft body, similar to the catheter head, inserting a small metal plate into a hole of the shaft body, or the like.

Here, the terminal provided at the shaft body for the connection with the wire of the catheter head may elongate in a coupling direction of the catheter head and the shaft body. For example, as shown in Fig. 11, the power supply terminal 2110, the temperature sensing terminal 2120 and/or the tactile sensing terminal 2130 may be formed to extend in a lateral direction of the shaft body. According to this embodiment of the present disclosure, when the catheter head and the shaft body are coupled, the power supply wire 300, the temperature sensing wire 620 and/or the tactile sensing wire 720 of the catheter head slides in along the coupling direction. Thus, if the terminal of the shaft body extends in the coupling direction, the contact between the terminal of the shaft body and the wire of the catheter head may be improved further.

Also, for this reason, the power supply wire 300, the temperature sensing wire 620 and/or the tactile sensing wire 720 of the catheter head may be formed to elongate along the coupling direction of the catheter head and the shaft body.

Preferably, a coupling guide member P may be provided to at least one of the catheter head and the shaft body to guide their coupling direction. For example, as shown in Figs. 15 and 17, a protrusion P1 may be formed at a distal end of the shaft body, and a groove P2 may be formed at a proximal end of the catheter head at a location corresponding to the protrusion P1 with a shape corresponding to the protrusion P1.

According to this embodiment of the present disclosure, when the catheter head 1000 is coupled to the shaft body 2000, a coupling direction may be guided. In particular, if at least one wire is formed at the catheter head 1000 and at least one terminal is formed at the shaft body 2000, when the catheter head 1000 and the shaft body 2000 are coupled, the wire and the terminal should be connected to each other. Therefore, in the above embodiment, since the coupling direction is guided by the coupling guide member P, the wire of the catheter head 1000 and the terminal of the shaft body 2000 may be easily and accurately coupled.

Further, various wires such as the power supply wire 300, the temperature sensing wire 620 and the tactile sensing wire 720 may be provided at the catheter head 1000, and in this case, various kinds of terminals may be formed at the shaft body 2000 to correspond to the wires. In this case, it is required to connect a wire and a terminal which mate with each other. Therefore, if the coupling guide member P is provided as in this embodiment, wires and terminals may be accurately coupled depending on their kinds.

Meanwhile, though not shown in the figures, the catheter according to the present disclosure may further include a cover provided at a distal end thereof In other words, even though it has been illustrated that the distal end of the cylinder member 100 is formed to have an open hollow, the distal end of the hollow may be closed by a cover.

The cover may be integrally configured with the cylinder member 100. For example, in the development view of Fig. 2, a circular cover may be provided at the right end of the cylinder member 100 to integrate with the cylinder member 100. In this case, the cylinder member 100 is bent circularly along the directions C1 and C2 of Fig. 2, and the circular cover may be coupled to the cylinder member 100 to seal the hollow at the right end of the cylinder member 100.

In other case, the cover may be provided separately from the cylinder member 100, and the cover may be coupled to the distal end of the cylinder member 100 in a state where the cylinder member 100 is bent into a circular shape.

Meanwhile, as in this embodiment, when the catheter according to the present disclosure includes the catheter head 1000 and the shaft body 2000, the distribution unit 400 may be located at the catheter head 1000 or the shaft body 2000. For example, the distribution unit 400 may be mounted to an inner space of the hollow shaft body 2000. According to this embodiment of the present disclosure, it is possible to prevent the catheter head 1000 from having a great size, by using the distribution unit 400, and other components may be introduced to the catheter head 1000 more easily. In addition, according to this embodiment of the present disclosure, the catheter head 1000 may have a simplified structure and thus be manufactured more easily.

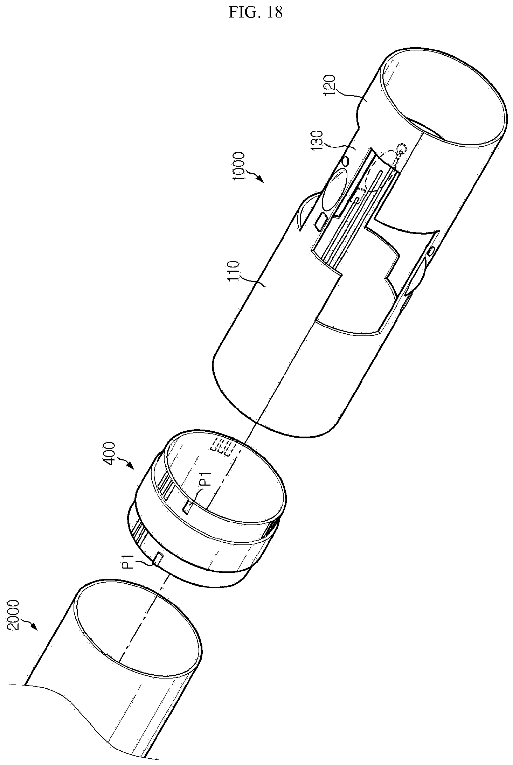

Fig. 18 is a perspective view schematically showing a catheter according to another embodiment of the present disclosure. The configurations of Fig. 18 similar to those of former embodiments will be not described in detail here, and the following explanation will be focused only on different features.

Referring to Fig. 18, the distribution unit 400 may be configured to have a hollow tube shape. At this time, the hollow of the distribution unit 400 may be coaxial with the hollow of the cylinder member 100. In addition, the distribution unit 400 may be coupled to one end of the cylinder member 100, particularly to the proximal end of the cylinder member 100 as shown in Fig. 18.

In addition, the shaft body 2000 may be coupled to the distribution unit 400. For example, as shown in Fig. 18, the shaft body 2000 may be coupled to the left side of the distribution unit 400, and the catheter head 1000 may be coupled to the right side thereof In this case, the distribution unit 400 may be configured to have a bushing form.

According to this embodiment of the present disclosure, it may be prevented that the size of the catheter head 1000 or the shaft body 2000 is increased by the distribution unit 400, and the distribution unit 400 may be utilized to mechanically couple the catheter head 1000 to the shaft body 2000.

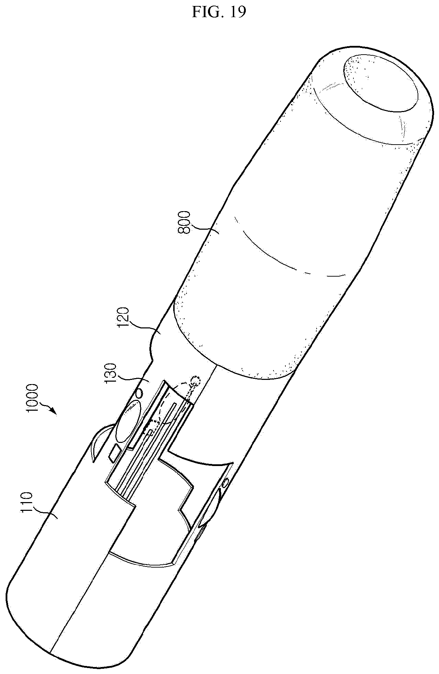

Fig. 19 is a perspective view schematically showing a catheter according to another embodiment of the present disclosure. The configurations of Fig. 19, similar to those of Figs. 1 to 18, will be not described in detail here, and the following explanation will be focused only on different features.

Referring to Fig. 19, the catheter according to the present disclosure may further include a terminal tip 800 at a front surface of a distal end of the catheter, namely a distal end of the catheter head.

The terminal tip 800 may be made of soft and flexible material. In particular, the terminal tip 800 may be made of a composition containing polyether block amide (PEBA). Here, additives other than polyether block amide may be further added to the composition for forming the terminal tip 800. For example, the terminal tip 800 may be made of a composition containing 70 weight% of polyether block amide and 30 weight% of barium sulfate, based on 100 weight% of the entire composition.

In this configuration of the present disclosure, when the distal end of the catheter moves along a blood vessel or the like, since the terminal tip 800 made of soft and flexible material is located at the front, the blood vessel is less damaged, and the moving direction of the catheter may be easily changed. Further, the terminal tip 800 made of soft and flexible material may be photographed using X-ray, and thus the location of the catheter head may be easily checked.

Preferably, the terminal tip 800 may have a hollow tube shape. In addition, the hollow of the terminal tip 800 may be formed to extend in the same direction as the longitudinal direction of the catheter. If the terminal tip 800 has a tube shape as described above, a guide wire may pass through the hollow of the terminal tip 800. For example, the terminal tip 800 may have a tube shape with a length of 6 mm and a hollow diameter of 0.7 mm.

The terminal tip 800 may be formed to elongate along the extending direction of the catheter. At this time, the terminal tip 800 may have different sizes depending on its longitudinal direction. In particular, if the terminal tip 800 has a cylindrical shape, the terminal tip may have a smallest diameter at its distal end other than other regions. For example, the distal end of the terminal tip 800 may have a diameter of 1.1 mm, which is smallest, when the thickest portion has a diameter of 1.3 mm.

The terminal tip 800 may have a suitable length, which is not too long or not too short. For example, the terminal tip 800 may have a length of 5 mm to 15 mm in the lateral direction of Fig. 19. In this configuration of the present disclosure, when moving along an inner space of a blood vessel or an inner space of a sheath, the terminal tip 800 may be easily move without being less disturbed. In addition, in the configuration of the present disclosure, a shape or the like of the blood vessel at a region where the terminal tip 800 is located may be easily found by checking bending and direction of the terminal tip 800.

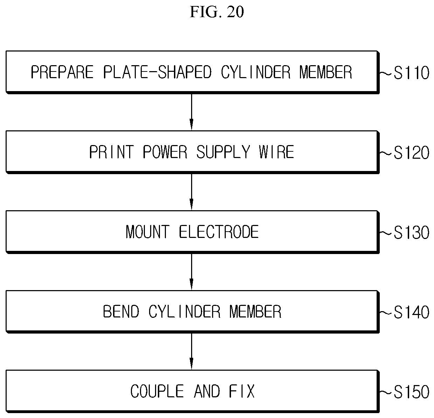

Fig. 20 is a schematic flowchart for illustrating a method for manufacturing a catheter according to an embodiment of the present disclosure.

Referring to Fig. 20, the catheter manufacturing method according to the present disclosure may include a cylinder member preparing step (S110), a power supply wire printing step (S120), an electrode mounting step (S130), a cylinder member bending step (S140) and a coupling and fixing step (S150).

In the cylinder member preparing step (S110), the plate-shaped cylinder member 100 is prepared as shown in Figs. 2, 5 and 10. The cylinder member 100 may have a planar shape widely spreading two-dimensionally, as described above.

In the power supply wire printing step (S120), the power supply wire 300 is printed on the cylinder member 100. For example, in Step S120, as shown in Figs. 2, 5 and 10, the power supply wire 300 may be printed by placing a conductor on one surface of the cylinder member 100 as a two-dimensional pattern.

In the electrode mounting step (S130), at least one electrode is mounted to the cylinder member 100. In particular, in Step S130, the electrode may be mounted to be connected to the power supply wire 300 of the cylinder member 100.

In addition, in Step S130, an electric conductive material for forming the electrode may be printed on the cylinder member.

In the cylinder member bending step (S140), the cylinder member 100 is bent to form a cylindrical shape with a hollow. For example, in Step S140, as indicated by C1 and C2 in Fig. 2, the plate-shaped cylinder member 100 is bent so that the cylinder member 100 has a cylindrical shape as shown in Fig. 1. Step S140 may be regarded as changing a two-dimensional configuration into a three-dimensional configuration.

In Step S140, the plate-shaped cylinder member 100 may be bent so that two portions of the cylinder member 100 spaced apart from each other approach each other. For example, in Step S140, as shown in Fig. 2, the plate-shaped cylinder member 100 may be bent so that the upper side A1 and the lower side A2 move close to each other.

In the coupling and fixing step (S150), the sides of the cylinder member 100 moving adj acent to each other by bending are coupled and fixed to each other. For example, in Step S150, the portion A of the configuration depicted in Fig. 1 is coupled and fixed so that the tube shape as shown in Fig. 1 may be maintained.

Here, in Step S150, a protrusion provided at one of the two sides of the cylinder member 100 moving adj acent to each other is inserted into an insert groove provided at the other of the two sides, so that the two sides are coupled and fixed to each other.

In other case, in Step S150, two sides of the cylinder member 100 moving adjacent to each other by bending may be adhered to each other by an adhesive so that the two sides are coupled and fixed to each other.

Meanwhile, in Step S150, a fixing force may be applied uniformly from one end of the hollow to the other end thereof, or a fixing force may be applied to a part of the region.

In addition, in the cylinder member preparing step (S110), the cylinder member 100 may include a first cylinder 110, a second cylinder 120 spaced apart from the first cylinder 110 by a predetermined distance, and a connection member 130 having one end connected to the first cylinder 110 and the other end connected to the second cylinder 120.

For example, in Step S110, the plate-shaped cylinder member 100 as shown in Fig. 5 may be prepared. In the configuration of Fig. 5, the plate-shaped first cylinder 110 may be a first substrate plate, and the plate-shaped second cylinder 120 may be a second substrate plate. In addition, the plate-shaped connection member 130 may be called a connection plate.

Also preferably, in the power supply wire printing step (S120), the power supply wire 300 may be printed from one end of the first cylinder 110 to a point of the connection member 130 at which the electrode is to be mounted. For example, as shown in Figs. 5 and 10, in Step S120, a power supply wire may be printed so that the power supply wire extends from the left end of the first cylinder 110 to a point where the electrode is mounted.

Also preferably, in the cylinder member preparing step (S110), the cylinder member 100 may include a plurality of connection members 130, and in the electrode mounting step (S130), electrodes may be mounted to at least two connection members 130, respectively. For example, in Step S110, as shown in Fig. 5, a cylinder member 100 having three connection members 130 may be prepared, and in Step S130, electrodes may be respectively mounted to the three connection members 130.

Also preferably, in the electrode mounting step (S130), the electrodes mounted to at least two connection members 130 may be spaced apart from each other by a predetermined distance in the longitudinal direction of the hollow formed in the bending step (S140). For example, as shown in Figs. 5 and 10, in Step S130, a plurality of electrodes may be mounted to the connection member 130 to be spaced apart from each other by a predetermined distance in a lateral direction.

Also preferably, in the cylinder member preparing step (S110), with respect to at least one of the first cylinder 110 and the second cylinder 120, a step or a slope may be formed in the longitudinal direction of the hollow formed in the bending step (S140) at a portion where the connection member 130 is connected. For example, in Step S110, the cylinder member may be prepared as shown in Fig. 10. In this case, the plate-shaped first cylinder 110 and/or the second cylinder 120 may have a step formed at a point where the connection member 130 is connected, as shown in the figures.

Also preferably, in Step S110, a plurality of connection members 130 may be spaced apart from each other by a predetermined distance in a direction perpendicular to the longitudinal direction of the hollow formed in Step S140. For example, in Step S110, as shown in Figs. 5 and 10, the cylinder member whose lateral direction is identical to the longitudinal direction of the hollow may be prepared so that a plurality of connection members 130 are spaced apart from each other by a predetermined distance in a vertical direction. In this case, if the cylinder member is bent so that its upper and lower sides are adjacent to each other, the hollow is formed in the lateral direction, and the connection members 130 may be spaced apart from each other based on the central axis of the hollow.

Meanwhile, the flowchart depicted in Fig. 20 is just an example, and the present disclosure is not limited thereto. For example, Step S130 may be performed before Step S120.

Also preferably, the catheter manufacturing method according to the present disclosure may further include mounting a distribution unit. For example, the catheter manufacturing method according to the present disclosure may further include a distribution unit mounting step between Step S130 and Step S140. In the distribution unit mounting step, as shown in Fig. 12, a distribution unit is mounted to the plate-shaped cylinder member to be connected to a plurality of power supply wires.

Also preferably, the catheter manufacturing method according to the present disclosure may further include printing a temperature sensing wire 620 on the plate-shaped cylinder member and mounting a temperature sensing member 610 to the cylinder member to be connected to the temperature sensing wire 620.

Here, the temperature sensing wire printing step and the temperature sensing member mounting step may be performed after Step S110 and before Step S140, but the present disclosure is not limited thereto.

Also preferably, the catheter manufacturing method according to the present disclosure may further include printing a tactile sensing wire 720 on the plate-shaped cylinder member and mounting a tactile sensing member 710 to the cylinder member to be connected to the tactile sensing wire 720.

Here, the tactile sensing wire printing step and the tactile sensing member mounting step may be performed after Step S110 and before Step S140, but the present disclosure is not limited thereto.

Meanwhile, if the catheter manufacturing method further includes the distribution unit mounting step, in the distribution unit mounting step, the distribution unit may be connected to a plurality of temperature sensing wires and/or a plurality of tactile sensing wires.

In addition, the catheter manufacturing method according to an embodiment of the present disclosure may further include printing a power input line 500, a temperature output line 630 and a tactile output line 730 on the plate-shaped cylinder member, similar to the power supply wire 300, the temperature sensing wire 620 and the tactile sensing wire 720, before Step S140.

Also preferably, in Step S140, the cylinder member may be bent circularly to have a cylindrical shape.

Also preferably, the catheter manufacturing method according to the present disclosure may further include preparing a shaft body as shown in Figs. 15 to 17, and after Step S150, may further include coupling the shaft body to the catheter head.

Also preferably, the catheter manufacturing method according to the present disclosure may further include preparing a terminal tip 800 as shown in Fig. 19, and after Step S150, may further include coupling the terminal tip 800 to the catheter head.

A denervation apparatus according to the present disclosure includes the catheter described above. In addition, the denervation apparatus may further include an energy supplying unit and an opponent electrode in addition to the catheter for denervation. Here, the energy supplying unit may be electrically connected to an electrode through the power supply wire. In addition, the opponent electrode may be electrically connected to the energy supplying unit through a cable or the like. In this case, the energy supplying unit may supply energy to the electrode of the catheter in the form of high frequency or the like, and the electrode of the catheter generates heat to ablate nerves around the blood vessel, thereby block the nerves.

The present disclosure has been described in detail. However, it should be understood that the detailed description and specific examples, while indicating preferred embodiments of the disclosure, are given by way of illustration only.

In addition, even though terms representing directions such as proximal, distal, upper, lower, right, left or the like have been used in the specification, the terms are just used to indicate relative locations for convenience and can be replaced with other words according to an observation point of an observer or an arrangement of a component, as obvious to those having ordinary skill in the art.

## Claims

1. A catheter, comprising:
a cylinder member (100) having a hollow (V) formed therein;
at least one electrode (200) mounted to the cylinder member (100) to generate heat; and
a power supply wire (300) printed on the cylinder member (100) and connected to the electrode (200) to give a power supply path for the electrode (200),
wherein the cylinder member (100) is configured by bending a plate-shaped member so that two sides (A1, A2) of the plate-shaped member spaced apart from each other along a longitudinal direction of the hollow (V) are coupled and fixed to each other,
wherein the electrode (200) and the power supply wire (300) having one end connected to the electrode (200) are provided in plural, and
wherein the catheter further comprises a distribution unit (400) to which at least two of the plurality of power supply wires (300) is connected and at least one power input line (500) is connected, so that the power supplied from a single power input line (500) is distributed to at least two power supply wire (300), and
wherein the distribution unit (400) is mounted to an inner wall of the cylinder member (100) and has a curved shape corresponding to the inner wall of the cylinder member (100).

2. The catheter according to claim1,
wherein one of the two sides (A1, A2) of the cylinder member (100) has a protrusion, the other of the two sides of the cylinder member has an insert groove, and the protrusion is inserted into the insert groove so that the two sides (A1, A2) are coupled and fixed to each other.

3. The catheter according to claim 1, wherein the cylinder member (100) includes:
a first cylinder (110) having a power supply wire (300) printed from one end thereof to the other end thereof;
a second cylinder (120) provided coaxially with the first cylinder (110) and spaced apart from the first cylinder (110) by a predetermined distance in the longitudinal direction of the hollow (V); and
a connection member (130) configured to have one end connected to the first cylinder (110) and the other end connected to the second cylinder (120), the electrode (200) being mounted to an outer surface of the connection member (130), the connection member (130) having a power supply wire (300) printed from one end thereof at least to a portion where the electrode (200) is mounted so as to be connected to the power supply wire (300) of the first cylinder (110).

4. The catheter according to claim 3,
wherein when a distance between the first cylinder (110) and the second cylinder (120) decreases, the connection member (130) is at least partially bent to form a bent portion, and the bent portion moves away from the hollow (V).

5. The catheter according to claim 3,
wherein the connection member (130) is provided in plural, and
wherein the electrode (200) is respectively mounted to at least two connection members (130).

6. The catheter according to claim 5,
wherein at least one of the first cylinder (110) and the second cylinder (120) has a step or a slope formed at a surface to which the connection member (130) is connected, in the longitudinal direction of the hollow (V).

7. The catheter according to claim 1,
wherein the distribution unit (400) is a multiplexer.

8. The catheter according to claim 1,
wherein the cylinder member (100) has a cylindrical shape.

9. The catheter according to claim 1,
wherein the distribution unit (400) is configured to be bendable.

10. The catheter according to claim 1, further comprising:
a temperature sensing member (610); and
a temperature sensing wire (620) printed on the cylinder member (100) and connected to the temperature sensing member (610) to transmit temperature information sensed by the temperature sensing member (610).

11. The catheter according to claim 1, further comprising:
a tactile sensing member (710); and
a tactile sensing wire (720) printed on the cylinder member (100) and connected to the tactile sensing member (710) to transmit tactile information sensed by the tactile sensing member (710).

12. The catheter according to claim 1, further comprising:
a shaft body (2000) formed to elongate in one direction and having an inner space formed along a longitudinal direction thereof, the shaft body (2000) being coupled to one end of the cylinder member (100).

13. The catheter according to claim 12,
wherein the shaft body (2000) has a power supply terminal (2110) contacting at least a part of the power supply wire (300) printed on the cylinder member.

14. The catheter according to claim 12,
wherein at least one of the cylinder member (100) and the shaft body (2000) includes a coupling guide member (P) configured to guide a coupling direction of the cylinder member (100) and the shaft body (2000).

15. The catheter according to claim 1, further comprising:
a terminal tip (800) coupled to the other end of the cylinder member (100).

16. A manufacturing method of a catheter, comprising:
preparing a plate-shaped cylinder member (100);
printing a power supply wire (300) on the plate-shaped cylinder member (100);
mounting an electrode (200) at the plate-shaped cylinder member (100) to be connected to the printed power supply wire (300);
mounting a distribution unit (400) at the plate-shaped cylinder member (100) to be connected to a plurality of power supply wires (300) and at least one power input line (500);
bending the cylinder member (100) so that two sides of the cylinder member (100) spaced apart from each other to get close to each other and thus the cylinder member (100) has a cylinder form with a hollow (V) therein; and
coupling and fixing the two sides (A1, A2) of the cylinder member (100), which have got close to each other by bending.

17. A denervation apparatus, comprising the catheter defined in claim 1.

## Patentansprüche

1. Katheter, umfassend:
ein Zylinderelement (100) mit einem darin ausgebildeten Hohlraum (V);
mindestens eine an dem Zylinderelement (100) montierte Elektrode (200) zum Erzeugen von Wärme; und
einen Stromversorgungsdraht (300), der auf das Zylinderelement (100) gedruckt und mit der Elektrode (200) zum Liefern eines Stromversorgungspfads für die Elektrode (200) verbunden ist,
wobei das Zylinderelement (100) durch Biegen eines plattenförmigen Elements, so dass zwei Seiten (A1, A2) des plattenförmigen Elements, die voneinander entlang einer Längsrichtung des Hohlraums (V) beabstandet sind, gekoppelt und aneinander fixiert sind, konfiguriert ist,
wobei die Elektrode (200) und der Stromversorgungsdraht (300) mit einem mit der Elektrode (200) verbundenen Ende mehrfach vorgesehen sind, und
wobei der Katheter ferner eine Verteileinheit (400) aufweist, mit der mindestens zwei der Vielzahl von Stromversorgungsdrähten (300) verbunden sind und mindestens eine Stromeingangsleitung (500) verbunden ist, so dass der von einer einzelnen Stromeingangsleitung (500) gelieferte Strom auf mindestens zwei Stromversorgunggeräte (300) verteilt wird, und
wobei die Verteileinheit (400) an einer Innenwand des Zylinderelements (100) montiert ist und eine mit der Innenwand des Zylinderelements (100) übereinstimmende gebogene Gestalt aufweist.

2. Katheter nach Anspruch 1, wobei eine der beiden Seiten (A1, A2) des Zylinderelements (100) einen Vorsprung aufweist, die andere der beiden Seiten des Zylinderelements eine Einsetznut aufweist und der Vorsprung in die Einsetznut so eingesetzt ist, dass die beiden Seiten (A1, A2) gekoppelt und aneinander fixiert sind.

3. Katheter nach Anspruch 1, wobei das Zylinderelement (100) enthält:
einen ersten Zylinder (110) mit einem Stromversorgungsdraht (300), der von einem Ende desselben zum anderen Ende desselben gedruckt ist;
einen zweiten Zylinder (120), der koaxial zum ersten Zylinder (110) vorgesehen und vom ersten Zylinder (110) um eine vorab festgelegte Entfernung in der Längsrichtung des Hohlraums (V) beabstandet ist; und
ein Verbindungselement (130), das konfiguriert ist, dass ein Ende mit dem ersten Zylinder (110) verbunden ist und das andere Ende mit dem zweiten Zylinder (120) verbunden ist, wobei die Elektrode (200) an einer Außenfläche des Verbindungselements (130) montiert ist, das Verbindungselement (130) einen Stromversorgungsdraht (300) aufweist, der von einem Ende desselben zumindest zu einem Abschnitt gedruckt ist, wo die Elektrode (200) montiert ist, um mit dem Stromversorgungsdraht (300) des ersten Zylinders (110) verbunden zu werden.

4. Katheter nach Anspruch 3, wobei, wenn eine Entfernung zwischen dem ersten Zylinder (110) und dem zweiten Zylinder (120) abnimmt, das Verbindungselement (130) zumindest teilweise gebogen wird, um einen gebogenen Abschnitt zu bilden, und sich der gebogene Abschnitt von dem Hohlraum (V) weg bewegt.

5. Katheter nach Anspruch 3, wobei das Verbindungselement (130) in einer Vielzahl vorgesehen ist, und wobei die Elektrode (200) an mindestens zwei Verbindungselemente (130) montiert ist.

6. Katheter nach Anspruch 5, wobei mindestens einer vom ersten Zylinder (110) und vom zweiten Zylinder (120) eine Stufe oder eine Schräge, die an einer Fläche ausgebildet ist, mit der das Verbindungselement (130) verbunden ist, in der Längsrichtung des Hohlraums (V) aufweist.

7. Katheter nach Anspruch 1, wobei die Verteileinheit (400) ein Multiplexer ist.

8. Katheter nach Anspruch 1, wobei das Zylinderelement (100) eine zylindrische Gestalt aufweist.

9. Katheter nach Anspruch 1, wobei die Verteileinheit (400) konfiguriert ist, um biegbar zu sein.

10. Katheter nach Anspruch 1, ferner umfassend:
ein Temperaturmesselement (610); und
einen Temperaturmessdraht (620), der auf das Zylinderelement (100) gedruckt und mit dem Temperaturmesselement (610) verbunden ist, um von dem Temperaturmesselement (610) erfasste Temperaturinformation zu senden.

11. Katheter nach Anspruch 1, ferner umfassend:
ein taktiles Sensorelement (710); und
einen taktilen Sensordraht (720), der auf das Zylinderelement (100) gedruckt und mit dem taktilen Sensorelement (710) verbunden ist, um von dem taktilen Sensorelement (710) erfasste taktile Information zu senden.

12. Katheter nach Anspruch 1, ferner umfassend:
einen Schaftkörper (2000), der in einer Richtung länglich ausgebildet ist und einen Innenraum aufweist, der entlang einer Längsrichtung desselben ausgebildet ist, wobei der Schaftkörper (1000) mit einem Ende des Zylinderelements (100) gekoppelt ist.

13. Katheter nach Anspruch 12, wobei der Schaftkörper (2000) einen Stromversorgungsanschluss (2110) aufweist, der zumindest einen Teil des auf das Zylinderelement gedruckten Stromversorgungsdrahts (300) kontaktiert.

14. Katheter nach Anspruch 12, wobei mindestens einer/eines vom Zylinderelement (100) und vom Schaftkörper (2000) ein Kopplungsführungselement (P) enthält, das konfiguriert ist, um eine Kopplungsrichtung des Zylinderelements (100) und des Schaftkörpers (2000) zu führen.

15. Katheter nach Anspruch 1, ferner umfassend:
eine Anschlussspitze (800), die mit dem anderen Ende des Zylinderelements (100) gekoppelt ist.

16. Verfahren zur Herstellung eines Katheters, umfassend:
Bereiten eines plattenförmigen Zylinderelements (100);
Drucken eines Stromversorgungsdrahts (300) auf das plattenförmige Zylinderelement (100);
Montieren einer Elektrode (200) an dem plattenförmigen Zylinderelement (100) zur Verbindung mit dem gedruckten Stromversorgungsdraht (300);
Montieren einer Verteileinheit (400) an das plattenförmige Zylinderelement (100) zum Verbinden mit einer Vielzahl von Stromversorgungsdrähten (300) und mindestens einer Stromeingangsleitung (500);
Biegen des Zylinderelements (100), so dass zwei Seiten des Zylinderelements (100), die voneinander beabstandet sind, nahe zusammenkommen und somit das Zylinderelement (100) eine Zylinderform mit einem Hohlraum (V) darin aufweist; und
Koppeln und Fixieren der beiden Seiten (A1, A2) des Zylinderelements (100), die durch Biegen nahe zusammengekommen sind.

17. Denervationsvorrichtung, umfassend den in Anspruch 1 definierten Katheter.

## Revendications

1. Cathéter, comprenant :
un élément de cylindre (100) ayant un creux (V) formé à l'intérieur de celui-ci ;
au moins une électrode (200) montée sur l'élément de cylindre (100) pour générer de la chaleur ; et
un fil de fourniture de puissance (300) imprimé sur l'élément de cylindre (100) et connecté à l'électrode (200) pour donner un chemin de fourniture de puissance à l'électrode (200),
dans lequel l'élément de cylindre (100) est configuré en pliant un élément en forme de plaque de sorte que deux côtés (A1, A2) de l'élément en forme de plaque espacés l'un de l'autre le long d'une direction longitudinale du creux (V) soient couplés et fixés l'un à l'autre,
dans lequel l'électrode (200) et le fil de fourniture de puissance (300) ayant une extrémité connectée à l'électrode (200) sont fournis en pluralité, et
dans lequel le cathéter comprend en outre une unité de distribution (400) à laquelle au moins deux de la pluralité de fils de fourniture de puissance (300) sont connectés et au moins une ligne d'entrée de puissance (500) est connectée, de sorte que la puissance fournie par une seule ligne d'entrée de puissance (500) est distribuée à au moins deux fils de fourniture de puissance (300), et
dans lequel l'unité de distribution (400) est montée sur une paroi intérieure de l'élément de cylindre (100) et a une forme incurvée correspondant à la paroi intérieure de l'élément de cylindre (100).

2. Cathéter selon la revendication 1, dans lequel l'un des deux côtés (A1, A2) de l'élément de cylindre (100) a une saillie, l'autre des deux côtés de l'élément de cylindre a une rainure d'insertion, et la saillie est insérée dans la rainure d'insertion de sorte que les deux côtés (A1, A2) soient couplés et fixés l'un à l'autre.

3. Cathéter selon la revendication 1, dans lequel l'élément de cylindre (100) comporte :
un premier cylindre (110) ayant un fil de fourniture de puissance (300) imprimé d'une extrémité de celui-ci à l'autre extrémité de celui-ci ;
un second cylindre (120) prévu coaxialement au premier cylindre (110) et espacé du premier cylindre (110) d'une distance prédéterminée dans la direction longitudinale du creux (V) ; et
un élément de connexion (130) configuré pour avoir une extrémité connectée au premier cylindre (110) et l'autre extrémité connectée au second cylindre (120), l'électrode (200) étant montée sur une surface extérieure de l'élément de connexion (130), l'élément de connexion (130) ayant un fil de fourniture de puissance (300) imprimé d'une extrémité de celui-ci à au moins une portion où l'électrode (200) est montée de manière à être connectée au fil de fourniture de puissance (300) du premier cylindre (110).

4. Cathéter selon la revendication 3, dans lequel lorsqu'une distance entre le premier cylindre (110) et le second cylindre (120) diminue, l'élément de connexion (130) est au moins partiellement plié pour former une portion pliée, et la portion pliée s'éloigne du creux (V) .

5. Cathéter selon la revendication 3, dans lequel l'élément de connexion (130) est prévu en une pluralité, et
dans lequel l'électrode (200) est montée respectivement sur au moins deux éléments de connexion (130) .

6. Cathéter selon la revendication 5, dans lequel au moins l'un du premier cylindre (110) et du second cylindre (120) a un gradin ou une pente formé(e) au niveau d'une surface à laquelle l'élément de connexion (130) est connecté, dans la direction longitudinale du creux (V).

7. Cathéter selon la revendication 1, dans lequel l'unité de distribution (400) est un multiplexeur.

8. Cathéter selon la revendication 1, dans lequel l'élément de cylindre (100) a une forme cylindrique.

9. Cathéter selon la revendication 1, dans lequel l'unité de distribution (400) est configurée pour être pliable.

10. Cathéter selon la revendication 1, comprenant en outre :
un élément de détection de température (610) ; et
un fil de détection de température (620) imprimé sur l'élément de cylindre (100) et connecté à l'élément de détection de température (610) pour transmettre des informations de température détectées par l'élément de détection de température (610).

11. Cathéter selon la revendication 1, comprenant en outre :
un élément de détection tactile (710) ; et
un fil de détection tactile (720) imprimé sur l'élément de cylindre (100) et connecté à l'élément de détection tactile (710) pour transmettre des informations tactiles détectées par l'élément de détection tactile (710).

12. Cathéter selon la revendication 1, comprenant en outre :
un corps d'arbre (2000) formé pour s'allonger dans une direction et ayant un espace intérieur formé le long d'une direction longitudinale de celui-ci, le corps d'arbre (2000) étant couplé à une extrémité de l'élément de cylindre (100).

13. Cathéter selon la revendication 12,
dans lequel le corps d'arbre (2000) a une borne de fourniture de puissance (2110) en contact avec au moins une partie du fil de fourniture de puissance (300) imprimé sur l'élément de cylindre.

14. Cathéter selon la revendication 12, dans lequel au moins l'un de l'élément de cylindre (100) et du corps d'arbre (2000) comporte un élément de guidage de couplage (P) configuré pour guider une direction de couplage de l'élément de cylindre (100) et du corps d'arbre (2000).

15. Cathéter selon la revendication 1, comprenant en outre :
une pointe terminale (800) couplée à l'autre extrémité de l'élément de cylindre (100).

16. Procédé de fabrication d'un cathéter, comprenant :
la préparation d'un élément de cylindre en forme de plaque (100) ;
l'impression d'un fil de fourniture de puissance (300) sur l'élément de cylindre en forme de plaque (100) ;
le montage d'une électrode (200) sur l'élément de cylindre en forme de plaque (100) à connecter au fil de fourniture de puissance imprimé (300) ;
le montage d'une unité de distribution (400) sur l'élément de cylindre en forme de plaque (100) à connecter à une pluralité de fils de fourniture de puissance (300) et à au moins une ligne d'entrée de puissance (500) ;
le pliage de l'élément de cylindre (100) de sorte que deux côtés de l'élément de cylindre (100) espacés l'un de l'autre se rapprochent l'un de l'autre et ainsi l'élément de cylindre (100) ait une forme de cylindre avec un creux (V) à l'intérieur de celui-ci ; et
le couplage et la fixation des deux côtés (A1, A2) de l'élément de cylindre (100), qui se sont rapprochés l'un de l'autre par pliage.

17. Appareil de dénervation, comprenant le cathéter défini dans la revendication 1.
